(19) Europäisches Patentamt
European Patent Office
Office européen des brevets

(11) EP 3 888 770 B1

(12) **EUROPEAN PATENT SPECIFICATION**

(45) Date of publication and mention of the grant of the patent:
**20.09.2023 Bulletin 2023/38**

(21) Application number: **19888519.6**

(22) Date of filing: **05.11.2019**

(51) International Patent Classification (IPC):
***B01D 1/22*** (2006.01) ***B01J 4/00*** (2006.01)
***C07C 231/12*** (2006.01) ***C07C 233/05*** (2006.01)
***F28D 3/02*** (2006.01) ***B01D 1/16*** (2006.01)

(52) Cooperative Patent Classification (CPC):
(C-Sets available)
**B01D 1/065; B01D 1/0064; B01D 1/007; B01D 1/16; B01D 1/22; C07C 231/12** (Cont.)

(86) International application number:
**PCT/JP2019/043186**

(87) International publication number:
**WO 2020/110615 (04.06.2020 Gazette 2020/23)**

(54) **RAW MATERIAL FEEDER AND N-VINYLCARBOXYLIC ACID AMIDE PRODUCTION METHOD**

ROHSTOFFZUFÜHRVORRICHTUNG UND VERFAHREN ZUR HERSTELLUNG VON N-VINYLCARBONSÄUREAMID

DISPOSITIF D’ALIMENTATION EN MATIÈRE PREMIÈRE ET PROCÉDÉ DE PRODUCTION D’AMIDE D’ACIDE N-VINYLCARBOXYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2018 JP 2018224144**

(43) Date of publication of application:
**06.10.2021 Bulletin 2021/40**

(73) Proprietor: **Resonac Corporation**
**Tokyo (JP)**

(72) Inventors:
- **TANAKA, Naoyuki**
  **Tokyo 105-8518 (JP)**
- **KAKO, Toshihiro**
  **Tokyo 105-8518 (JP)**
- **SEITOH, Masaaki**
  **Tokyo 105-8518 (JP)**
- **KOBAYASHI, Takamitsu**
  **Tokyo 105-8518 (JP)**

(74) Representative: **Strehl Schübel-Hopf & Partner**
**Maximilianstrasse 54**
**80538 München (DE)**

(56) References cited:
**JP-A- H04 352 748**
**JP-A- S55 162 304**
**JP-A- S60 228 894**
**JP-A- 2006 284 166**
**JP-A- 2007 507 329**
**JP-A- 2013 212 995**
**JP-A- 2017 211 094**
**US-A- 5 496 448**
**US-A- 6 058 623**
**US-A1- 2008 193 645**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 888 770 B1

(52) Cooperative Patent Classification (CPC): (Cont.)

C-Sets
**C07C 231/12, C07C 233/05**

## Description

### Technical Field

The present invention relates to a raw material feeder to distribute and feed a liquid compound as a raw material to a plurality of evaporation tubes of a falling film evaporator, and a method of producing N-vinylcarboxylic acid amide in which N-(1-alkoxyethyl)carboxylic acid amide is used as a raw material.

### Background Art

In relation to methods of producing N-vinylcarboxylic acid amide, a large number of methods have been proposed (see PTL 1, for example). In a production method described in PTL 1, a method of producing N-vinylcarboxylic acid amide involving evaporating and thermally decomposing a raw material of N-(2-alkoxyethyl)carboxylic acid amide is disclosed.

Thus, in the method of producing N-vinylcarboxylic acid amide by thermal decomposition, an important factor for stable operation is to stably evaporate the raw material in an evaporator and to introduce the material into a thermal decomposition reactor. Therefore, to stably evaporate the raw material, a multi-tube falling film evaporator comprising a plurality of evaporation tubes is typically used as the evaporator.

For feeding of the raw material to the evaporator and distribution of the raw material to the plurality of evaporation tubes, a distributor (a distribution plate) system and a spray nozzle system are typically used, but these systems require a corresponding raw material feeding speed. If the speed is less than or equal to the feeding speed at which this performance can be maintained, raw material dispersibility drops. If the raw material dispersibility drops, for example, one-sided flow of the raw material occurs to hinder sufficient evaporation, and a liquid raw material flows into the thermal decomposition reactor. The inflow of the liquid raw material into the thermal decomposition reactor at a high temperature might generate tar or solid aggregates (referred to also as coagulum). The generation of coagulum results in a problem of the thermal decomposition reactor being blocked, thereby making it difficult to perform the stable operation. A further example of a raw material feeder is disclosed in PTL2.

### Citation List

#### Patent Literature

PTL 1: WO 2017/002494 (A1)
PTL2:US5496448

### Summary of Invention

#### Technical Problem

An object of the present invention is to solve the above problems and provide a raw material feeder and a method of producing N-vinylcarboxylic acid amide in which a raw material is fed to a plurality of evaporation tubes of a falling film evaporator with a stable raw material dispersibility, so that a problem of a thermal decomposition reactor being blocked due to coagulum can be inhibited, and an operation can be stably and continuously performed for a long period of time.

#### Solution to Problem

The present invention relates to the following.

A raw material feeder to distribute and feed a liquid compound as a raw material to a plurality of evaporation tubes of a falling film evaporator, the raw material feeder comprising: a main body section comprising a plurality of communication holes facing the plurality of evaporation tubes, a restriction orifice housed in an interior of each of the communication holes, and comprising an orifice hole, a liquid feeding tip housed to face the restriction orifice on an upstream side of the restriction orifice in the interior of the communication hole, and comprising a hole portion facing the orifice hole, a first O-ring fitted into a side surface of the liquid feeding tip from outside, and sandwiched between the side surface of the liquid feeding tip and an inner surface of the communication hole, and a second O-ring sandwiched between the liquid feeding tip and the restriction orifice so that the orifice hole is present in an inner region. The raw

material feeder further comprises: a nozzle housed to face the restriction orifice on a downstream side of the restriction orifice in an interior of the communication hole, and comprising a nozzle hole facing the orifice hole, and a third O-ring sandwiched between the nozzle and the restriction orifice so that the orifice hole is present in an inner region, wherein the nozzle hole houses a liquid diffuser in an interior, and wherein the nozzle hole comprises an upstream hole portion on an upstream side, and a downstream hole portion communicating with a downstream end of the upstream hole portion, and having a diameter increasing toward a lower end of the nozzle, and the liquid diffuser is housed in the upstream hole portion.

In a preferred embodiment the main body section comprises a first flange comprising a first hole portion, and a second flange facing a surface of the first flange on a downstream side, and comprising a second hole portion facing the first hole portion, the first hole portion and the second hole portion form the communication hole, the second hole portion comprises a large diameter hole on an upstream side, and a small diameter hole communicating with the large diameter hole, and having a diameter smaller than a diameter of the large diameter hole, a side portion of the nozzle on the upstream side is a protrusion protruding outward, and the protrusion abuts on a bottom surface of the large diameter hole on the downstream side.

The invention further includes a method of producing N-vinylcarboxylic acid amide comprising: a feeding step of feeding, by the claimed raw material feeder, N-(1-alkoxyethyl)carboxylic acid amide as a raw material to a falling film evaporator, an evaporation step of evaporating, by the falling film evaporator, the raw material, to form a vaporized raw material, and a thermal decomposition step of feeding the vaporized raw material to a thermal decomposition reactor, to thermally decompose the raw material under reduced pressure. Further preferred embodiments are defined in dependent claims.

Advantageous Effects of Invention

According to the present invention, a raw material feeder and a method of producing N-vinylcarboxylic acid amide can be provided in which a raw material is fed to a plurality of evaporation tubes of a falling film evaporator with a stable raw material dispersibility, so that a problem of a thermal decomposition reactor being blocked due to coagulum can be inhibited, and an operation can be stably and continuously performed for a long period of time.

Brief Description of Drawings

[Fig. 1] Fig. 1 is a schematic cross-sectional view (an overall view) of a raw material feeder according to an embodiment of the present invention.

[Fig. 2] Fig. 2 is a schematic cross-sectional view (an enlarged view) of the raw material feeder according to the embodiment of the present invention.

[Fig. 3] Fig. 3 is a cross-sectional view in an A-A direction of Fig. 2.

[Fig. 4] Fig. 4 is a schematic overall diagram of a production device to produce N-vinylcarboxylic acid amide of the present invention.

[Fig. 5] Fig. 5 is a flowchart showing a method of producing N-vinylcarboxylic acid amide of the present invention.

[Fig. 6] Fig. 6 is a schematic cross-sectional view (an overall view) of a conventional raw material feeder shown as a comparative example.

[Fig. 7] Fig. 7 is a schematic cross-sectional view (an enlarged view) of the conventional raw material feeder shown as the comparative example. Description of Embodiments

A raw material feeder according to an embodiment of the present invention is, as shown in Fig. 1, a raw material feeder to distribute and feed a liquid compound as a raw material to a plurality of evaporation tubes 8 of a falling film evaporator. The raw material feeder comprises a main body section 51 comprising a plurality of communication holes 50 facing the plurality of evaporation tubes 8, a restriction orifice 7 housed in an interior of each of the communication holes 50 comprising an orifice hole 7a, a liquid feeding tip 6 housed to face the restriction orifice 7 on an upstream side of the restriction orifice 7 in the interior of the communication hole 50, comprising a hole portion 6a facing the orifice hole 7a, a first O-ring 10 fitted into a side surface of the liquid feeding tip 6 from outside, sandwiched between the side surface of the liquid feeding tip 6 and an inner surface of the communication hole 50, and a second O-ring 11 sandwiched between the liquid feeding tip 6 and the restriction orifice 7 so that the orifice hole 7a is present in an inner region. The claimed methods concern feeding a raw material wherein the liquid compound is N-(1-alkoxyethyl)carboxylic acid amide.

In the side surface of the liquid feeding tip 6, a first O-ring groove may be provided in which the first O-ring 10 is to be mounted. Furthermore, a surface of the liquid feeding tip 6 that faces the restriction orifice 7 may be provided with a second O-ring groove in which the second O-ring 11 is to be mounted.

The main body section 51 comprises a first flange 2 and a second flange 3, and further comprises a feeding flange 1 comprising a raw material feeding port 1a to feed the raw material, the feeding flange 1 being provided on the first flange 2.

The first O-ring 10 inhibits the raw material from flowing through a space between the side surface of the liquid feeding tip 6 and the inner surface of the communication hole 50. The second O-ring 11 inhibits the raw material from flowing through a space between the liquid feeding tip 6 and the restriction orifice 7. That is, the first O-ring 10 and the second O-ring 11 inhibit the raw material so that the raw material flows only through the orifice hole 7a.

Examples of a material of the first O-ring 10 and the second O-ring 11 include ethylene propylene diene rubber (EPDM) and silicone rubber, from viewpoints of resistance to the raw material, heat resistance and durability.

The communication holes 50 as many as the evaporation tubes 8 to feed the raw material are prepared. A diameter of a downstream end of the communication hole 50 is determined in accordance with a tube diameter of each of the evaporation tubes 8 to which the raw material is fed.

A hole diameter of the orifice hole 7a provided in the restriction orifice 7 may be set to an optimum hole diameter in accordance with a flow velocity of the raw material to be distributed through the evaporation tubes 8 and a number of the evaporation tubes 8 for use.

The hole diameter of the orifice hole 7a is set so that a flow rate coefficient $\alpha$ calculated with the following equation (1) is preferably from 0.50 to 1.00, more preferably from 0.60 to 0.85, and further preferably from 0.65 to 0.75.

$$\text{Flow rate coefficient } \alpha = Q/(A\times(2\times\Delta P/\rho)^{0.5}) \qquad (1)$$

In Equation (1), "Q" is a volumetric flow rate ($m^3/s$) that means a volume of a fluid flowing through the orifice hole 7a per unit time, "A" is a cross-sectional area ($m^2$) of the orifice hole 7a, "$\Delta P$" is pressure loss (Pa) per unit flow rate of the fluid passing through the orifice hole 7a, and "$\rho$" is a density ($kg/m^3$) of the raw material passing through the orifice hole 7a.

For a purpose of decreasing an error in hole diameter of the orifice hole 7a, the flow rate coefficient $\alpha$ is measured in advance to prepare a required number of orifice holes 7a having less variations, so that the raw material can be dispersed more evenly.

A range of a flow velocity of the raw material to be distributed through the orifice hole 7a can be set to be more than or equal to a range in which a differential pressure before and after the distribution of the raw material through the orifice hole 7a is not zero, and a range less than or equal to a feeding pressure upper limit. The range of the flow velocity of the raw material to be distributed through the orifice hole 7a can be set in accordance with the hole diameter of the orifice hole 7a. Therefore, from a viewpoint that the flow velocity meets operation conditions, the hole diameter of the orifice hole 7a is preferably from 0.10 to 1.0 mm, more preferably from 0.15 to 0.9 mm, and further preferably from 0.20 to 0.8 mm.

As shown in Fig. 1, the raw material feeder according to the embodiment of the present invention comprises a nozzle 5 housed to face the restriction orifice 7 on a downstream side of the restriction orifice 7 in an interior of the communication hole 50, comprising a nozzle hole 5a facing the orifice hole 7a, and a third O-ring 12 sandwiched between the nozzle 5 and the restriction orifice 7 so that the orifice hole 7a is present in an inner region.

In a surface of the nozzle 5 that faces the restriction orifice 7, a third O-ring groove may be provided in which the third O-ring 12 is to be mounted.

The third O-ring 12 inhibits the raw material from flowing through a space between the nozzle 5 and the restriction orifice 7. That is, the third O-ring 12 inhibits the raw material so that the raw material flows only through the orifice hole 7a.

As a material of the third O-ring 12, the same material as the above material of the first O-ring 10 and second O-ring 11 may be used.

As shown in Fig. 1 and Fig. 2, the nozzle hole 5a is configured to house a liquid diffuser 30 in an interior. There are not any special restrictions on fixing means for the liquid diffuser 30. Examples of the fixing means include means for holding the liquid diffuser 30 with an inner wall surface of the nozzle hole 5a to fix the diffuser and means for integrally forming the liquid diffuser 30 in the nozzle hole 5a to fix the diffuser. The liquid diffuser 30 is formed in a shape with a gap between the diffuser and the nozzle 5, as shown in Fig. 3, in order to diffuse the raw material distributed through the orifice hole 7a and distribute the raw material along an inner wall surface of the nozzle 5. There are not any special restrictions on a cross-sectional shape of the liquid diffuser 30 as long as the diffuser is formed in the shape with the gap between the diffuser and the nozzle 5. The diffuser may have a quadrangular shape shown in Fig. 3, a triangular shape, a polygonal shape, or the like.

As shown in Fig. 2, the nozzle hole 5a comprises an upstream hole portion 5b on an upstream side and a downstream hole portion 5c communicating with a downstream end of the upstream hole portion 5b and having a diameter increasing toward a lower end of the nozzle 5, and the liquid diffuser 30 is housed in the upstream hole portion 5b. According to this configuration, the liquid diffuser 30 diffuses the raw material distributed through the orifice hole 7a

in the upstream hole portion 5b, and further diffuses the raw material in the downstream hole portion 5c having the diameter increasing toward the lower end of the nozzle 5, so that the raw material can be uniformly and stably distributed to an inner wall surface of the evaporation tube 8.

As shown in Fig. 1 and Fig. 2, the main body section 51 comprises the first flange 2 comprising a first hole portion 2a, and the second flange 3 facing a surface of the first flange 2 on a downstream side comprising a second hole portion 3a communicating with the first hole portion 2a, and the first hole portion 2a and the second hole portion 3a form the communication hole 50. A configuration is preferable in which the second hole portion 3a comprises a large diameter hole 3b on the upstream side, and a small diameter hole 3c communicating with the large diameter hole 3b, having a diameter smaller than a diameter of the large diameter hole 3b, a side portion of the nozzle 5 on the upstream side is a protrusion 5d protruding outward, and the protrusion 5d abuts on a bottom surface of the large diameter hole 3b on the downstream side. According to this configuration, the nozzle 5 can be stably installed in the second flange 3.

A circumferential surface of the first hole portion 2a of the first flange 2 on the downstream side may be cut and provided with a female thread, and a circumferential surface of the liquid feeding tip 6 on the downstream side may be cut and provided with a male thread. Consequently, the liquid feeding tip 6 can be screwed to the first hole portion 2a. In this case, the first O-ring 10 may be mounted to a side surface of the liquid feeding tip 6 on the upstream side that is not cut or provided with the male screw and may be sandwiched between this side surface and a circumferential surface of the first hole portion 2a of the first flange 2 on the upstream side that is not cut or provided with the female screw.

It is preferable that, as shown in Fig. 2, the downstream end of the nozzle 5 extends to a downstream side of a downstream end of the second flange 3. Consequently, the downstream end of the nozzle 5 can extend into the evaporation tube 8 of an evaporator 4, and the raw material can be securely guided into the evaporation tube 8.

Hereinafter, description will be made as to an installation method of the raw material feeder according to the embodiment of the present invention with reference to Fig. 1.

First, the feeding flange 1, the first flange 2 and the second flange 3 are prepared.

Next, the liquid feeding tip 6, the first O-ring 10 and the second O-ring 11 are prepared, and the first O-ring 10 and the second O-ring 11 are mounted to the liquid feeding tip 6. Then, the liquid feeding tip 6 to which the first O-ring 10 and the second O-ring 11 are mounted is disposed in the first hole portion 2a of the first flange 2.

The nozzle 5 and the third O-ring 12 are next prepared, and the third O-ring 12 is mounted to the nozzle 5. Then, the nozzle 5 to which the third O-ring 12 is mounted is disposed in the second hole portion 3a of the second flange 3.

The restriction orifice 7 is then prepared and the restriction orifice 7 is disposed on the nozzle 5 disposed in the second flange 3.

Next, the first flange 2 is disposed on the second flange 3. The arranged first flange 2 and second flange 3 are fixed with a fixing member such as a bolt (not shown). At this time, it is preferable to seal a space between the first flange 2 and the second flange 3 with an O-ring in order to improve close contact therebetween.

The liquid feeding tip 6 is then tightened by using a tool corresponding to a tool engagement portion 6b such as a hexagonal hole provided in the hole portion 6a of the liquid feeding tip 6 on an upstream side to seal the restriction orifice 7.

Subsequently, the feeding flange 1 is disposed on the first flange 2. The arranged feeding flange 1, first flange 2 and second flange 3 are fixed with a fixing member 20 such as a bolt to form the raw material feeder according to the embodiment of the present invention. At this time, it is preferable to seal a space between the feeding flange 1 and the first flange 2 by use of an O-ring in order to improve close contact therebetween.

Next, the raw material feeder is disposed in the evaporator 4 comprising a flange comprising a plurality of communication holes and the plurality of evaporation tubes 8 mounted to the communication holes. When disposing the raw material feeder, positions of the communication holes of the raw material feeder are matched with positions of the communication holes of the evaporator 4. Then, the arranged raw material feeder and evaporator 4 are fixed with fixing members 21 such as bolts to complete installation. At this time, it is preferable to seal a space between the raw material feeder (the second flange 3) and the evaporator 4 by use of a spiral gasket in order to improve close contact therebetween.

Fig. 4 shows a schematic overall diagram of a production device comprising a falling film evaporator in which the raw material feeder according to the embodiment of the present invention is installed.

The production device shown in Fig. 4 comprises a raw material tank 100, a raw material feeder 101, an evaporator (the falling film evaporator) 102, a thermal decomposition reactor 103, a reaction solution receiver 104, and a reaction solution storage tank 105.

In the evaporator 102 and the thermal decomposition reactor 103, a temperature adjustment device, for example, configured to distribute heat medium through a jacket structure is provided, and temperatures of the evaporator 102 and the thermal decomposition reactor 103 can be adjusted with the temperature adjustment device.

In the raw material tank 100, a pressure adjustment device such as a pressure pump is provided, and pressures of the raw material tank 100 and the raw material feeder 101 (the upstream side of the restriction orifice 7) can be adjusted on pressurizing conditions with the pressure adjustment device.

In the reaction solution receiver 104, a pressure adjustment device such as a pressure pump is provided, and

pressures of the raw material feeder 101 (the downstream side of the restriction orifice 7), the evaporator 102, the thermal decomposition reactor 103 and the reaction solution receiver 104 can be adjusted on decompression conditions with the pressure adjustment device.

As shown in Fig. 5, a method of producing N-vinylcarboxylic acid amide according to the embodiment of the present invention comprises a feeding step S1, an evaporation step S2, and a thermal decomposition step S3. Hereinafter, the method of producing N-vinylcarboxylic acid amide according to the embodiment of the present invention will be described in detail.

(Feeding step S1)

The feeding step S1 is a step of feeding, by the raw material feeder according to the embodiment of the present invention, a liquid compound of N-(1-alkoxyethyl)carboxylic acid amide as the raw material to the evaporator 4.

In the feeding step S1, raw material liquid fed from the raw material feeding port 1a is fed through the feeding flange 1 to the first flange 2. Gas (air) mixed in the raw material during the liquid feeding is discharged through a gas vent 9. In the first flange 2, the raw material liquid is distributed to all installed liquid feeding tips 6 and fed to the restriction orifices 7 of the second flange 3. The raw material liquid passed through the restriction orifices 7 is introduced at an almost even flow velocity into the respective evaporation tubes 8 of the evaporator 4.

In the feeding step S 1, a feeding speed of the raw material to the evaporator is preferably from 5 to 350 kg/h, more preferably from 10 to 150 kg/h, and further preferably from 15 to 80 kg/h. The feeding speed of the raw material to the evaporator is in the above range, so that raw material dispersibility to the plurality of the evaporation tubes of the evaporator can be maintained, and the raw material can be stably evaporated.

In the feeding step S1, the raw material feeder feeds the raw material to each of the plurality of evaporation tubes at a flow rate variation coefficient of preferably 10.0% or less, more preferably 9.0% or less, and further preferably 8.0% or less. An upper limit of the variation coefficient of the flow rate is the above numeric value, so that the raw material dispersibility to the plurality of evaporation tubes of the evaporator can be maintained, and the raw material can be stably evaporated.

Note that as for the variation coefficient of the flow rate, the flow rate of the raw material fed to each of the plurality of evaporation tubes in a certain time is measured, and a standard deviation to an average value of flow rates in all the evaporation tubes is calculated. The coefficient indicates a degree of flow rate variation obtained from the standard deviation. Specifically, the variation coefficient of the flow rate is a coefficient calculated with the standard deviation ÷ the average value × 100(%).

In the feeding step S1, the raw material feeder feeds the raw material to each of the plurality of evaporation tubes with a flow rate error of preferably - 20% or more and +30% or less, more preferably -15% or more and +25% or less, and further preferably -10% or more and +20% or less. The error of the flow rate is in the above range, so that the raw material dispersibility to the plurality of evaporation tubes of the evaporator can be maintained, and the raw material can be stably evaporated.

Note that as for the error of the flow rate, the flow rate of the raw material fed to each of the plurality of evaporation tubes in a certain time is measured, and a relative error to the average value of the flow rates in all the evaporation tubes is calculated.

In the feeding step S1, the raw material feeder feeds the raw material to each of the plurality of evaporation tubes at a flow velocity of preferably 2.0 g/min or more and 80.0 g/min or less, more preferably 2.5 g/min or more and 78.0 g/min or less, and further preferably 3.0 g/min or more and 76.0 g/min or less. The flow velocity is in the above range, so that the raw material dispersibility to the plurality of evaporation tubes of the evaporator can be maintained, and the raw material can be stably evaporated.

In the method of producing N-vinylcarboxylic acid amide according to the embodiment of the present invention, as the raw material, preferably N-(1-alkoxyethyl)carboxylic acid amide represented by the following general formula (I) is used.

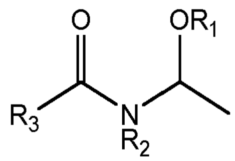

(I)

wherein $R_1$ represents a $C_1$ to $C_5$ alkyl group, $R_2$ represents a hydrogen atom or a $C_1$ to $C_5$ alkyl group, and $R_3$ represents a $C_1$ to $C_5$ alkyl group.

Examples of N-(1-alkoxyethyl)carboxylic acid amide include N-(1-methoxyethyl)acetamide, N-(1-methoxyethyl)-N-methylacetamide, N-(1-ethoxyethyl)acetamide, N-(1-ethoxyethyl)-N-methylacetamide, N-(1-propxyethyl)acetamide, N-(1-isopropoxyethyl)acetamide, N-(1-butoxyethyl)acetamide, N-(1-isobutoxyethyl)acetamide, N-(1-methoxyethyl)propionamide, N-(1-ethoxyethyl)propionamide, N-(1-propoxyethyl)propionamide, N-(1-isopropoxyethyl)propionamide, N-(1-buthoxyethyl)propionamide, N-(1-isobuthoxyethyl)propionamide, N-(1-methoxyethyl)isobutylamide, N-(1-ethoxyethyl)isobutylamide, N-(1-propoxyethyl)isobutylamide, N-(1-isopropoxyethyl)isobutylamide, N-(1-buthoxyethyl)isobutylamide and N-(1-isobuthoxyethyl)isobutylamide. The examples preferably include N-(1-methoxyethyl)acetamide, N-(1-isopropxyethyl)acetamide and N-(1-methoxyethyl)isobutylamide, and more preferably include N-(1-methoxyethyl)acetamide.

(Evaporation Step S2)

The evaporation step S2 is a step of evaporating, by the evaporator, the raw material, to form a vaporized raw material.

The evaporation step S2 can be performed under reduced pressure or under normal pressure and is preferably performed under reduced pressure. Specifically, the evaporation step S2 is performed with a reaction pressure preferably from 1 to 30 kPa, more preferably from 3 to 25 kPa, and further preferably from 5 to 20 kPa.

The evaporation step S2 is required to be performed specifically at a temperature to evaporate and vaporize the raw material, preferably from 100 to 200°C, more preferably from 110 to 190°C, and further preferably from 120 to 180°C.

(Thermal Decomposition Step S3)

The thermal decomposition step S3 is a step of feeding the vaporized raw material to a thermal decomposition reactor to thermally decompose the raw material. There are not any special restrictions on flow of the vaporized raw material to be fed to the thermal decomposition reactor as long as the material flows into the thermal decomposition reactor. The flow may be ascending flow or descending flow.

The thermal decomposition step S3 can be performed under reduced pressure or under normal pressure and is preferably performed under reduced pressure. Specifically, the thermal decomposition step S3 is performed with a reaction pressure preferably from 1 to 30 kPa, more preferably from 3 to 25 kPa, and further preferably from 5 to 20 kPa.

From a viewpoint of efficiently performing thermal decomposition, the thermal decomposition step S3 is performed specifically at a temperature preferably from 250 to 500°C, more preferably from 300 to 480°C, and further preferably from 350 to 450°C.

From a viewpoint of securely performing the thermal decomposition, a staying time in the thermal decomposition step S3 is preferably from 0.1 to 10 seconds, more preferably from 0.2 to 9 seconds, and further preferably from 0.3 to 8 seconds.

From the viewpoint of efficiently performing the thermal decomposition, it is preferable that the thermal decomposition reactor has a multi-tube structure.

A reactant thermal decomposed in the thermal decomposition step S3 is cooled to be liquefied and sent to a reaction solution receiver. The reactant stored in the reaction solution receiver is sent to and stored in a reaction solution storage tank.

Preferably, N-vinylcarboxylic acid amide obtained by the above steps is represented by the following general formula (II) and corresponds to N-(1-alkoxyethyl)carboxylic acid amide that is a suitable raw material represented by the general formula (I).

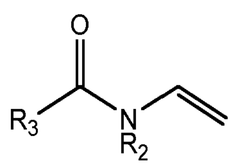

(II)

wherein $R_2$ represents a hydrogen atom or $C_1$ to $C_5$ alkyl group, and $R_3$ represents a $C_1$ to $C_5$ alkyl group.

Examples of N-vinylcarboxylic acid amide include N-vinylacetamide, N-methyl-N-vinylacetamide, N-vinylpropionamide, N-methyl-N-vinylpropionamide, N-vinylisobutylamide and N-methyl-N-vinylisobutylamide, and preferably include N-vinylacetamide.

Examples

Hereinafter, the present invention will be further specifically described by way of examples, and the present

invention is not limited to the following examples without departing from the scope.

Example 1

A raw material feeder shown in Fig. 1 was installed in a shell and tube evaporator comprising 22 evaporation tubes (tubes) each having a diameter of 25.4 mm and a length of 2500 mm. A restriction orifice of the raw material feeder was prepared in a hole diameter of 0.25 mm. A flow rate coefficient $\alpha$ of the restriction orifice was measured, and 22 restriction orifices having a flow rate coefficient $\alpha$ of 0.7 were selected and incorporated in the raw material feeder.
At a speed of 167 g/min for 5 minutes, N-(1-methoxyethyl)acetamide was continuously fed to the raw material feeder to carry out distribution test.
As a result of calculation of variation of a flow rate in each evaporation tube from an amount of N-(1-methoxyethyl)acetamide collected from each evaporation tube, relative to an average value, an error was from -10 to +19%, a standard deviation $\sigma$ was 2.7, and a variation coefficient was 7.2%.

Example 2

A device similar to that of Example 1 was used, and N-(1-methoxyethyl)acetamide was continuously fed to a raw material feeder at a speed of 833 g/min for 5 minutes to carry out distribution test.
As a result of calculation of variation of a flow rate in each evaporation tube from an amount of N-(1-methoxyethyl)acetamide collected from each evaporation tube, relative to an average value, an error was from -11 to +17%, a standard deviation $\sigma$ was 14.5, and a variation coefficient was 7.7%.

Comparative Example 1

A conventional raw material feeder shown in Figs. 6, 7 was installed in the same shell and tube evaporator as in Example 1. The conventional raw material feeder is different from the raw material feeder shown in Fig. 1 in that a first O-ring 10 and a third O-ring 12 are not provided. A restriction orifice of the conventional raw material feeder was prepared in a hole diameter of 0.25 mm. A flow rate coefficient $\alpha$ of the restriction orifice was measured, and 22 restriction orifices having the flow rate coefficient $\alpha$ of 0.7 were selected and incorporated in the conventional raw material feeder.
At a speed of 833 g/min for 5 minutes, N-(1-methoxyethyl)acetamide was continuously fed to the conventional raw material feeder to carry out distribution test.
As a result of calculation of variation of a flow rate in each evaporation tube from an amount of N-(1-methoxyethyl)acetamide collected from each evaporation tube, relative to an average value, an error was from -76 to +25%, a standard deviation $\sigma$ was 44.1, and a variation coefficient was 23.3%.

Comparative Example 2

A distributor type raw material distribution device was installed in the same shell and tube evaporator as in Example 1. In the distributor type raw material distribution device, 22 holes each having $\phi$2 mm were provided in correspondence to respective evaporation tubes.
At a speed of 833 g/min for 5 minutes, N-(1-methoxyethyl)acetamide was continuously fed to the distributor type raw material distribution device to carry out distribution test. N-(1-methoxyethyl)acetamide was collected only from two evaporation tubes and was not evenly distributed or fed.

Table 1

| | Example 1 | | Example 2 | | Comparative Example 1 | | Comparative Example 2 | |
|---|---|---|---|---|---|---|---|---|
| Evaporation tube | Mass (g) | Error of flow rate (%) | Mass (g) | Error of flow rate (%) | Mass (g) | Error of flow rate (%) | Mass (g) | Error of flow rate (%) |
| 1 | 43.5 | +15 | 194.7 | +3 | 228.2 | +20 | 3166.7 | +1572 |
| 2 | 37.2 | -2 | 189.4 | 0 | 45.5 | -76 | 0 | -100 |
| 3 | 37.2 | -2 | 184.1 | -3 | 217.5 | +15 | 1000 | +428 |
| 4 | 38.8 | +2 | 184.1 | -3 | 216.4 | +14 | 0 | -100 |
| 5 | 37.2 | -2 | 189.4 | 0 | 182.8 | -3 | 0 | -100 |

(continued)

| | Example 1 | | Example 2 | | Comparative Example 1 | | Comparative Example 2 | |
|---|---|---|---|---|---|---|---|---|
| Evaporation tube | Mass (g) | Error of flow rate (%) | Mass (g) | Error of flow rate (%) | Mass (g) | Error of flow rate (%) | Mass (g) | Error of flow rate (%) |
| 6 | 35.7 | -6 | 178.9 | -6 | 236.4 | +25 | 0 | -100 |
| 7 | 37.2 | -2 | 184.1 | -3 | 189.8 | 0 | 0 | -100 |
| 8 | 41.9 | +11 | 215.7 | +14 | 205.7 | +9 | 0 | -100 |
| 9 | 37.2 | -2 | 184.1 | -3 | 194.5 | +3 | 0 | -100 |
| 10 | 35.7 | -6 | 199.9 | +6 | 110.5 | -42 | 0 | -100 |
| 11 | 37.2 | -2 | 194.7 | +3 | 207.1 | +9 | 0 | -100 |
| 12 | 41.9 | +11 | 189.4 | 0 | 194.5 | +3 | 0 | -100 |
| 13 | 38.8 | +2 | 178.9 | -6 | 184.9 | -2 | 0 | -100 |
| 14 | 35.7 | -6 | 173.6 | -8 | 190.9 | +1 | 0 | -100 |
| 15 | 34.1 | -10 | 168.4 | -11 | 135.0 | -29 | 0 | -100 |
| 16 | 35.7 | -6 | 173.6 | -8 | 146.8 | -23 | 0 | -100 |
| 17 | 45.0 | +19 | 221.0 | +17 | 208.4 | +10 | 0 | -100 |
| 18 | 35.7 | -6 | 173.6 | -8 | 182.8 | -3 | 0 | -100 |
| 19 | 35.7 | -6 | 210.4 | +11 | 225.0 | +19 | 0 | -100 |
| 20 | 37.2 | -2 | 215.7 | +14 | 229.3 | +21 | 0 | -100 |
| 21 | 37.2 | -2 | 184.1 | -3 | 205.4 | +8 | 0 | -100 |
| 22 | 37.2 | -2 | 178.9 | -6 | 229.3 | +21 | 0 | -100 |
| Average | 37.9 | - | 189.4 | - | 189.4 | - | 189.4 | - |
| Standard deviation $\sigma$ | 2.7 | - | 14.5 | - | 44.1 | - | 682.2 | - |
| Variation coefficient (%) | 7.2 | - | 7.7 | - | 23.3 | - | 360.2 | - |

By use of a raw material feeder of the present invention, for example, one-sided flow of a raw material does not occur even at a speed less than or equal to a feeding speed at which performance of a distributor (distribution plate) system can be maintained. That is, by use of the raw material feeder of the present invention, even at a low raw material feeding speed in production of N-vinylcarboxylic acid amide, the raw material can be fed with stabilized raw material dispersibility to a plurality of evaporation tubes of a falling film evaporator, a problem of a thermal decomposition reactor being blocked due to coagulum can be inhibited, and a continuous operation can be stably performed for a long period of time.

Industrial Applicability

According to the present invention, N-vinylcarboxylic acid amide obtained in a production method in which a raw material feeder is used is a useful monomer in production of N-vinylcarboxylic acid amide polymer to be used in a coagulant, a liquid absorbent, a thickener, or the like, and additionally in various industrial applications.

Reference Signs List

| | |
|---|---|
| 1 | feeding flange |
| 1a | raw material feeding port |
| 2 | first flange |
| 2a | first hole portion |
| 3 | second flange |
| 3a | second hole portion |
| 3b | large diameter hole |
| 4 | evaporator |
| 5 | nozzle |
| 5a | nozzle hole |
| 5b | upstream hole portion |
| 5c | downstream hole portion |
| 5d | protrusion |
| 6 | liquid feeding tip |
| 6a | hole portion |
| 6b | tool engagement portion |
| 7 | restriction orifice |
| 7a | orifice hole |
| 8 | evaporation tube |
| 9 | gas vent |
| 10 | first O-ring |
| 11 | second O-ring |
| 12 | third O-ring |
| 20, 21 | fixing member |
| 30 | liquid diffuser |
| 50 | communication hole |
| 51 | main body section |
| 100 | raw material tank |
| 101 | raw material feeder |
| 102 | evaporator |
| 103 | thermal decomposition reactor |
| 104 | reaction solution receiver |
| 105 | reaction solution storage tank |

## Claims

**1.** A raw material feeder (101) to distribute and feed a liquid compound as a raw material to a plurality of evaporation tubes (8) of a falling film evaporator (102), the raw material feeder (101) comprising:

a main body section (51) comprising a plurality of communication holes (50) facing the plurality of evaporation tubes (8),
a restriction orifice (7) housed in an interior of each of the communication holes (50), and comprising an orifice hole (7a),
a liquid feeding tip (6) housed to face the restriction orifice (7) on an upstream side of the restriction orifice (7) in the interior of the communication hole (50), and comprising a hole portion (6a) facing the orifice hole (7a),
a first O-ring (10) fitted into a side surface of the liquid feeding tip (6) from outside, and sandwiched between the side surface of the liquid feeding tip (6) and an inner surface of the communication hole (50),
a second O-ring (11) sandwiched between the liquid feeding tip (6) and the restriction orifice (7) so that the orifice hole (7a) is present in an inner region,
a nozzle (5) housed to face the restriction orifice (7) on a downstream side of the restriction orifice (7) in an interior of the communication hole (50), and comprising a nozzle hole (5a) facing the orifice hole (7a) and housing a liquid diffuser (30) in an interior, wherein the nozzle hole (5a) comprises an upstream hole portion (5b) on an upstream side, and a downstream hole portion (5c) communicating with a downstream end of the upstream hole portion (5b), and having a diameter increasing toward a lower end of the nozzle, and the liquid diffuser (30) is housed in the upstream hole portion (5b), and
a third O-ring (12) sandwiched between the nozzle (5) and the restriction orifice (7) so that the orifice hole (7a) is present in an inner region.

2. The raw material feeder according to claim 1, wherein the main body section comprises a first flange (2) comprising a first hole portion (2a), and a second flange (3) facing a surface of the first flange (2) on a downstream side, and comprising a second hole portion (3a) facing the first hole portion (2a),

   the first hole portion (2a) and the second hole portion (3a) form the communication hole (50),
   the second hole portion (3a) comprises a large diameter hole (3b) on an upstream side, and a small diameter hole (3c) communicating with the large diameter hole (3b), and having a diameter smaller than a diameter of the large diameter hole (3b),
   a side portion of the nozzle (5) on the upstream side is a protrusion (5d) protruding outward, and
   the protrusion (5d) abuts on a bottom surface of the large diameter hole (3b) on the downstream side.

3. A method of producing N-vinylcarboxylic acid amide comprising:

   a feeding step of feeding, by the raw material feeder (101) according to claim 1 or 2, N-(1-alkoxyethyl)carboxylic acid amide as a raw material to a falling film evaporator (102),
   an evaporation step of evaporating, by the falling film evaporator (102), the raw material, to form a vaporized raw material, and
   a thermal decomposition step of feeding the vaporized raw material to a thermal decomposition reactor, to thermally decompose the raw material under reduced pressure.

4. The method of producing N-vinylcarboxylic acid amide according to claim 3, wherein the evaporation step is performed under reduced pressure.

5. The method of producing N-vinylcarboxylic acid amide according to claim 3 or 4, wherein the thermal decomposition step is performed under reduced pressure.

6. The method of producing N-vinylcarboxylic acid amide according to any one of claims 3 to 5, wherein the raw material feeder feeds the raw material to each of the plurality of evaporation tubes with a flow rate variation coefficient of 10.0% or less.

7. The method of producing N-vinylcarboxylic acid amide according to any one of claims 3 to 6, wherein the raw material feeder feeds the raw material to each of the plurality of evaporation tubes with a flow rate error of -20% or more and +30% or less.

8. The method of producing N-vinylcarboxylic acid amide according to any one of claims 3 to 7, wherein the raw material feeder feeds the raw material to each of the plurality of evaporation tubes at a flow velocity of 2.0 g/min or more and 80.0 g/min or less.

9. The method of producing N-vinylcarboxylic acid amide according to any one of claims 3 to 8, wherein the N-(1-alkoxyethyl)carboxylic acid amide is N-(1-methoxyethyl) acetamide.

**Patentansprüche**

1. Rohmaterialzuführung (101) zum Verteilen und Zuführen einer flüssigen Verbindung als ein Rohmaterial zu einer Vielzahl von Verdampfungsrohren (8) eines Fallfilmverdampfers (102), wobei die Rohmaterialzuführung (101) umfasst:

   einen Hauptkörperabschnitt (51) mit einer Vielzahl von Verbindungslöchern (50), die der Vielzahl von Verdampfungsrohren (8) zugewandt sind,
   eine Begrenzungsöffnung (7), die in einem Innenraum jedes der Verbindungslöcher (50) untergebracht ist und ein Öffnungsloch (7a) umfasst,
   eine Flüssigkeitszuführungsspitze (6), die so untergebracht ist, dass sie der Begrenzungsöffnung (7) auf einer stromaufwärts gelegenen Seite der Begrenzungsöffnung (7) im Inneren des Verbindungslochs (50) gegenüberliegt, und die einen Lochabschnitt (6a) umfasst, der dem Öffnungsloch (7a) gegenüberliegt,
   einen ersten O-Ring (10), der von außen in eine Seitenfläche der Flüssigkeitszuführungsspitze (6) eingepasst ist und zwischen der Seitenfläche der Flüssigkeitszuführungsspitze (6) und einer Innenfläche des Verbindungslochs (50) liegt,

einen zweiten O-Ring (11), der zwischen der Flüssigkeitszufuhrspitze (6) und der Begrenzungsöffnung (7) angeordnet ist, so dass sich das Öffnungsloch (7a) in einem inneren Bereich befindet,
eine Düse (5), die so untergebracht ist, dass sie der Begrenzungsöffnung (7) auf einer stromabwärtigen Seite der Begrenzungsöffnung (7) in einem Innenraum des Verbindungslochs (50) zugewandt ist, und ein Düsenloch (5a) umfasst, das dem Öffnungsloch (7a) zugewandt ist und einen Flüssigkeitsdiffusor (30) in einem Innenraum aufnimmt, wobei das Düsenloch (5a) einen stromaufwärts gelegenen Lochabschnitt (5b) auf einer stromaufwärts gelegenen Seite und einen stromabwärts gelegenen Lochabschnitt (5c) umfasst, der mit einem stromabwärts gelegenen Ende des stromaufwärts gelegenen Lochabschnitts (5b) in Verbindung steht und einen Durchmesser aufweist, der zu einem unteren Ende der Düse hin zunimmt, und der Flüssigkeitsdiffusor (30) in dem stromaufwärts gelegenen Lochabschnitt (5b) untergebracht ist, und
einen dritten O-Ring (12), der zwischen der Düse (5) und der Begrenzungsöffnung (7) angeordnet ist, so dass sich die Öffnungsloch (7a) in einem inneren Bereich befindet.

**2.** Rohmaterialzuführung nach Anspruch 1, wobei der Hauptkörperabschnitt einen ersten Flansch (2) mit einem ersten Lochabschnitt (2a) und einen zweiten Flansch (3) umfasst, der einer Oberfläche des ersten Flansches (2) auf einer stromabwärtigen Seite gegenüberliegt und einen zweiten Lochabschnitt (3a) umfasst, der dem ersten Lochteil (2a) gegenüberliegt,

der erste Lochabschnitt (2a) und der zweite Lochabschnitt (3a) das Kommunikationsloch (50) bilden,
der zweite Lochabschnitt (3a) ein Loch (3b) mit großem Durchmesser auf einer stromaufwärts gelegenen Seite und ein Loch (3c) mit kleinem Durchmesser umfasst, das mit dem Loch (3b) mit großem Durchmesser in Verbindung steht und einen Durchmesser aufweist, der kleiner als ein Durchmesser des Lochs (3b) mit großem Durchmesser ist,
ein Seitenabschnitt der Düse (5) auf der stromaufwärts gelegenen Seite ein nach außen ragender Vorsprung (5d) ist, und
der Vorsprung (5d) an einer Bodenfläche des Lochs (3b) mit großem Durchmesser auf der stromabwärts gelegenen Seite anliegt.

**3.** Verfahren zur Herstellung von N-Vinylcarbonsäureamid, umfassend:

einen Zuführungsschritt der Zuführung von N-(1-Alkoxyethyl)carbonsäureamid als Rohmaterial zu einem Fallfilmverdampfer (102) durch die Rohmaterialzuführung (101) nach Anspruch 1 oder 2,
einen Verdampfungsschritt, bei dem das Rohmaterial durch den Fallfilmverdampfer (102) verdampft wird, um ein verdampftes Rohmaterial zu bilden, und
einen thermischen Zersetzungsschritt, bei dem das verdampfte Rohmaterial einem thermischen Zersetzungsreaktor zugeführt wird, um das Rohmaterial unter reduziertem Druck thermisch zu zersetzen.

**4.** Verfahren zur Herstellung von N-Vinylcarbonsäureamid nach Anspruch 3, wobei der Verdampfungsschritt unter vermindertem Druck durchgeführt wird.

**5.** Verfahren zur Herstellung von N-Vinylcarbonsäureamid nach Anspruch 3 oder 4, wobei der thermische Zersetzungsschritt unter vermindertem Druck durchgeführt wird.

**6.** Verfahren zur Herstellung von N-Vinylcarbonsäureamid nach einem der Ansprüche 3 bis 5, wobei die Rohmaterialzuführung das Rohmaterial in jedes der mehreren Verdampfungsrohre mit einem Durchflussvariationskoeffizienten von 10,0 % oder weniger einspeist.

**7.** Verfahren zur Herstellung von N-Vinylcarbonsäureamid nach einem der Ansprüche 3 bis 6, wobei die Rohmaterialzuführung das Rohmaterial jedem der mehreren Verdampfungsrohre mit einem Durchflussfehler von -20% oder mehr und +30% oder weniger zuführt.

**8.** Verfahren zur Herstellung von N-Vinylcarbonsäureamid nach einem der Ansprüche 3 bis 7, wobei die Rohmaterialzuführung das Rohmaterial in jedes der mehreren Verdampfungsrohre mit einer Fließgeschwindigkeit von 2,0 g/min oder mehr und 80,0 g/min oder weniger einspeist.

**9.** Verfahren zur Herstellung von N-Vinylcarbonsäureamid nach einem der Ansprüche 3 bis 8, wobei das N-(1-Alkoxyethyl)carbonsäureamid N-(1-methoxyethyl)acetamid ist.

## Revendications

1. Dispositif d'alimentation en matière première (101) pour distribuer et fournir un composé liquide en tant que matière première à une pluralité de tubes d'évaporation (8) d'un évaporateur à film tombant (102), le dispositif d'alimentation en matière première (101) comprenant :

    une section de corps principal (51) comprenant une pluralité de trous de communication (50) étant face à la pluralité de tubes d'évaporation (8),
    un orifice de restriction (7) logé dans un intérieur de chacun des trous de communication (50), et comprenant un trou d'orifice (7a),
    une pointe d'alimentation en liquide (6) logée afin d'être face à l'orifice de restriction (7) sur un côté en amont de l'orifice de restriction (7) dans l'intérieur du trou de communication (50), et comprenant une portion de trou (6a) étant face au trou d'orifice (7a),
    un premier joint torique (10) ajusté dans une surface latérale de la pointe d'alimentation en liquide (6) à partir de l'extérieur, et pris en sandwich entre la surface latérale de la pointe d'alimentation en liquide (6) et une surface interne du trou de communication (50),
    un deuxième joint torique (11) pris en sandwich entre la pointe d'alimentation en liquide (6) et l'orifice de restriction (7) de sorte que le trou d'orifice (7a) est présent dans une région interne,
    une buse (5) logée afin d'être face à l'orifice de restriction (7) sur un côté en aval de l'orifice de restriction (7) dans un intérieur du trou de communication (50), et comprenant un trou de buse (5a) étant face au trou d'orifice (7a) et logeant un diffuseur de liquide (30) dans un intérieur, dans lequel le trou de buse (5a) comprend une portion de trou en amont (5b) sur un côté en amont, et une portion de trou en aval (5c) communiquant avec une extrémité en aval de la portion de trou en amont (5b), et ayant un diamètre augmentant vers une extrémité inférieure de la buse, et le diffuseur de liquide (30) est logé dans la portion de trou en amont (5b), et
    un troisième joint torique (12) pris en sandwich entre la buse (5) et l'orifice de restriction (7) de sorte que le trou d'orifice (7a) est présent dans une région interne.

2. Dispositif d'alimentation en matière première selon la revendication 1, dans lequel la section de corps principal comprend une première bride (2) comprenant une première portion de trou (2a), et une seconde bride (3) étant face à une surface de la première bride (2) sur un côté en aval, et comprenant une seconde portion de trou (3a) étant face à la première portion de trou (2a),

    la première portion de trou (2a) et la seconde portion de trou (3a) forment le trou de communication (50),
    la seconde portion de trou (3a) comprend un trou de grand diamètre (3b) sur un côté en amont, et un trou de petit diamètre (3c) communiquant avec le trou de grand diamètre (3b), et ayant un diamètre plus petit qu'un diamètre du trou de grand diamètre (3b),
    une portion latérale de la buse (5) sur le côté en amont est une saillie (5d) faisant saillie vers l'extérieur, et
    la saillie (5d) vient en butée sur une surface inférieure du trou de grand diamètre (3b) sur le côté en aval.

3. Procédé de production d'amide d'acide N-vinylcarboxylique comprenant :

    une étape d'alimentation consistant à fournir, par le dispositif d'alimentation en matière première (101) selon la revendication 1 ou 2, un amide d'acide N-(1-alcoxyéthyl)carboxylique en tant que matière première à un évaporateur à film tombant (102),
    une étape d'évaporation consistant à évaporer, par l'évaporateur à film tombant (102), la matière première, pour former une matière première vaporisée, et
    une étape de décomposition thermique consistant à fournir la matière première vaporisée à un réacteur de décomposition thermique, pour décomposer thermiquement la matière première sous pression réduite.

4. Procédé de production d'amide d'acide N-vinylcarboxylique selon la revendication 3, dans lequel l'étape d'évaporation est effectuée sous pression réduite.

5. Procédé de production d'amide d'acide N-vinylcarboxylique selon la revendication 3 ou 4, dans lequel l'étape de décomposition thermique est effectuée sous pression réduite.

6. Procédé de production d'amide d'acide N-vinylcarboxylique selon l'une quelconque des revendications 3 à 5, dans lequel le dispositif d'alimentation en matière première fournit la matière première à chacun de la pluralité de tubes d'évaporation avec un coefficient de variation de débit de 10,0 % ou moins.

7. Procédé de production d'amide d'acide N-vinylcarboxylique selon l'une quelconque des revendications 3 à 6, dans lequel le dispositif d'alimentation en matière première fournit la matière première à chacun de la pluralité de tubes d'évaporation avec une erreur de débit de -20 % ou plus et +30 % ou moins.

8. Procédé de production d'amide d'acide N-vinylcarboxylique selon l'une quelconque des revendications 3 à 7, dans lequel le dispositif d'alimentation en matière première fournit la matière première à chacun de la pluralité de tubes d'évaporation à une vitesse d'écoulement de 2,0 g/min ou plus et 80,0 g/min ou moins.

9. Procédé de production d'amide d'acide N-vinylcarboxylique selon l'une quelconque des revendications 3 à 8, dans lequel l'amide d'acide N-(1-alcoxyéthyl)carboxylique est du N-(1-méthoxyéthyl)acétamide.

[Fig. 1]

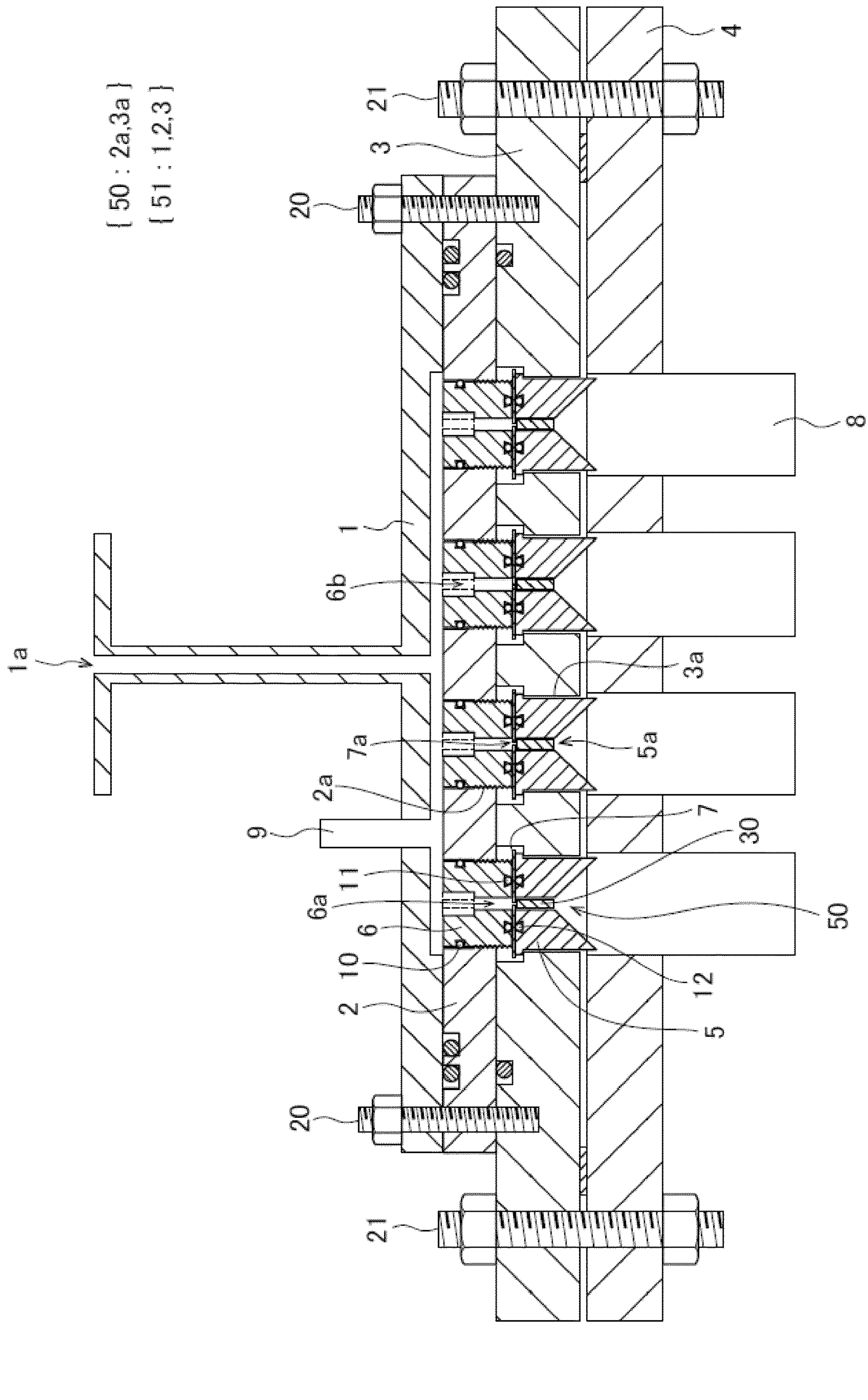
{ 50 : 2a,3a }
{ 51 : 1,2,3 }
4
21
3
20
8
1
6b
1a
3a
5a
7a
2a
9
7
30
11
6a
6
50
10
12
2
5
20
21

[Fig. 2]

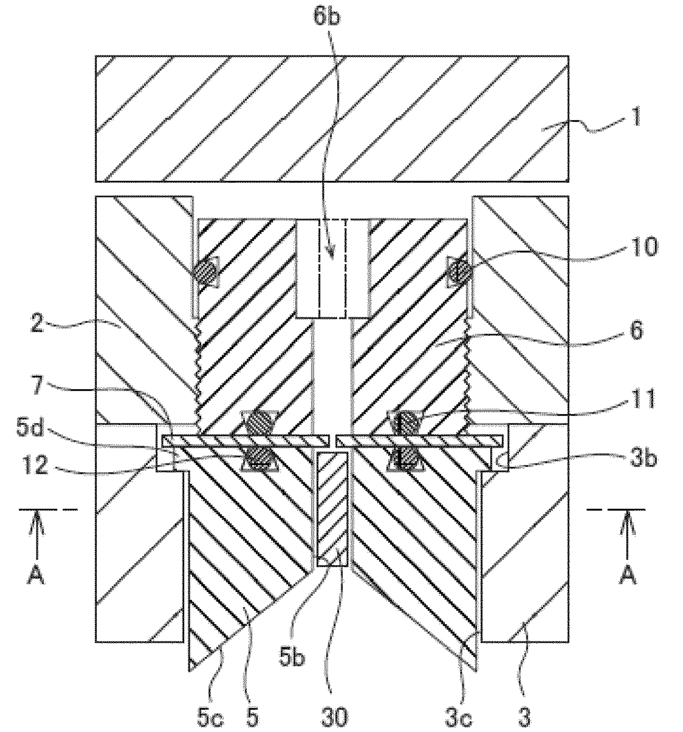


{ 5a : 5b,5c }
{ 3a : 3b,3c }

[Fig. 3]

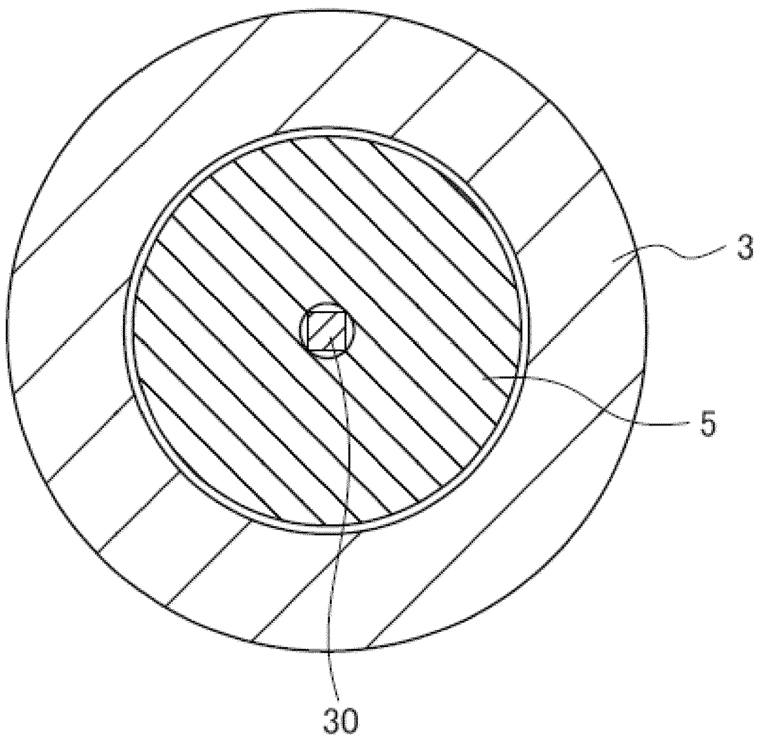

[Fig. 4]

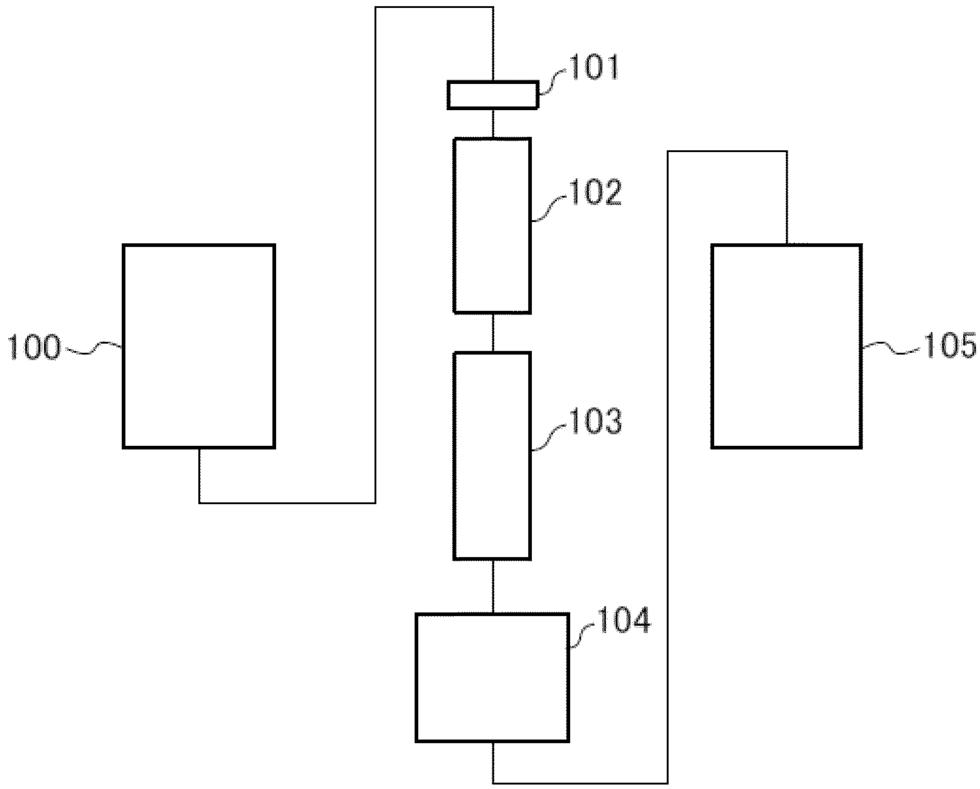
101
102
100
105
103
104

[Fig. 5]

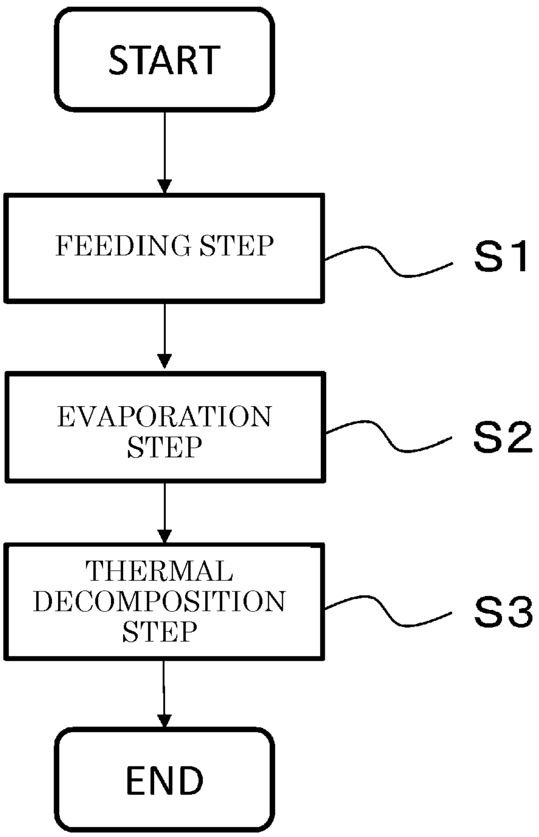
START
FEEDING STEP
S1
EVAPORATION STEP
S2
THERMAL DECOMPOSITION STEP
S3
END

[Fig. 6]

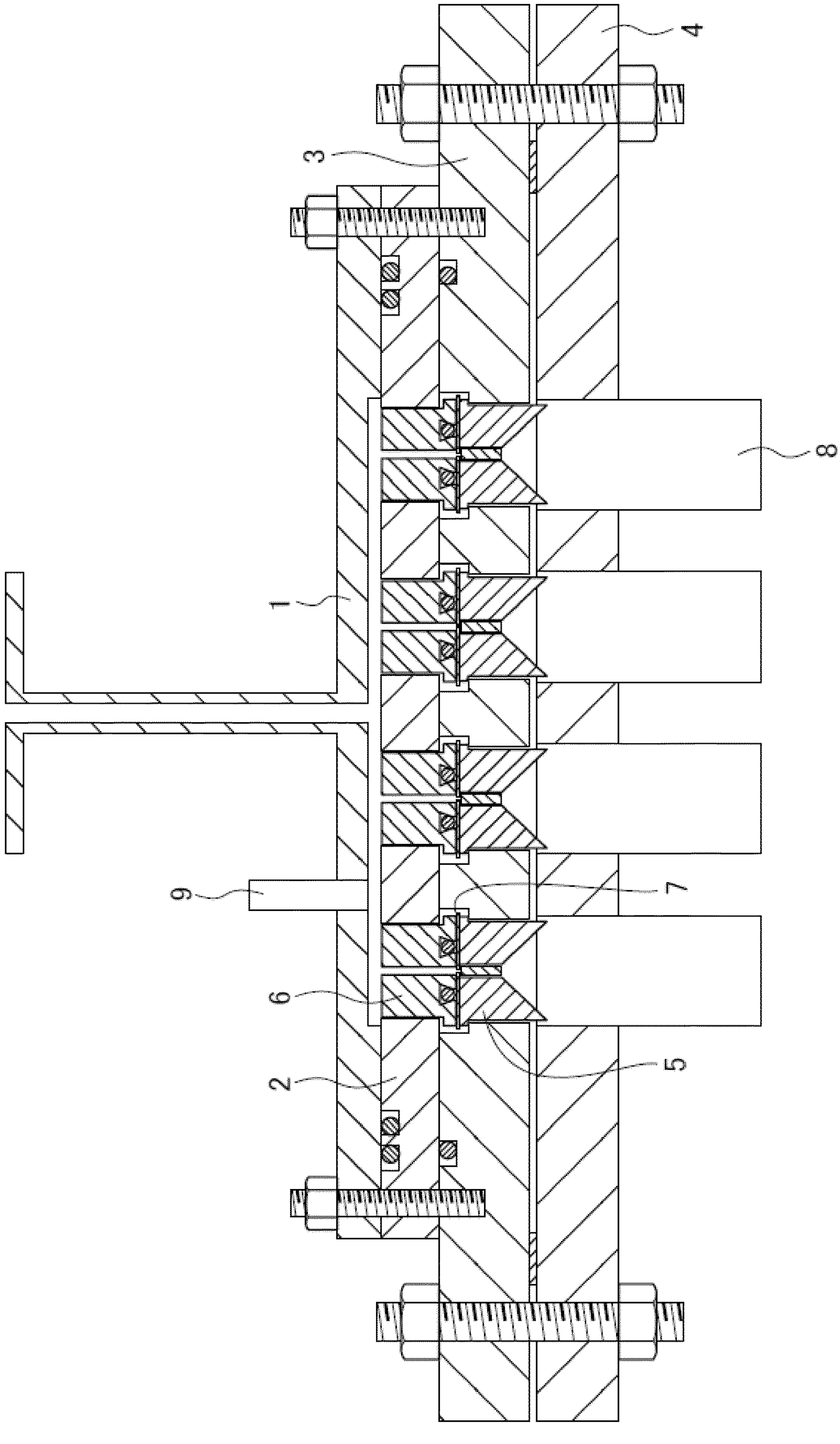

[Fig. 7]

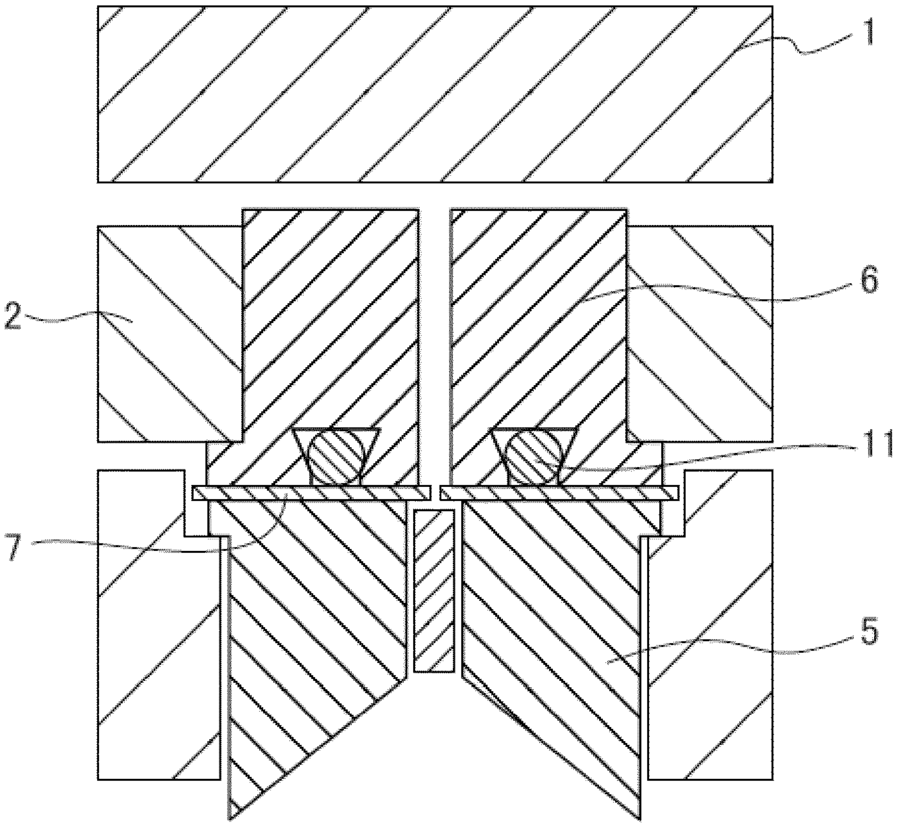

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017002494 A1
- US 5496448 A